# EUROPEAN PATENT APPLICATION

(11) **EP 4 298 933 A1**
(43) Date of publication of application: **03.01.2024**
(21) Application number: 23180748.8
(22) Date of filing: 21.06.2023
(51) Int. Cl.: A24F 40/465

(54) **HEATING MECHANISM COMPRISING AN ELECTROMAGNETIC HEATING COIL**

(30) Priority: 30.06.2022 CN 202210755773
(71) Applicant: Shenzhen Smoore Technology Limited, Shenzhen Guangdong 518102 (CN)
(72) Inventor: LUO, Yongjie, SHENZHEN, 518102 (CN); YANG, Baomin, SHENZHEN, 518102 (CN); FAN, Jichang, SHENZHEN, 518102 (CN)
(74) Representative: Corradini, Corrado

(57) **Abstract**

The present disclosure relates to a heating mechanism (100) and an electronic vaporization device. The heating mechanism (100) includes: a heating element (10) configured in a longitudinal shape, the heating element (10) being provided with an accommodating cavity (11) extending along its longitudinal direction; and an electromagnetic heating coil (30) arranged around the heating element (10). In the longitudinal direction of the heating element (10), the axial center of the electromagnetic heating coil (30) is misaligned with the longitudinal center of the accommodating cavity (11). After the electromagnetic heating coil is energized, a heating electric field with the strongest magnetic induction can be formed at a position corresponding to its own axial center. For an aerosol-generating substrate in the accommodating cavity, the high-temperature field can be distributed to the top, so that the aerosol-generating substrate can be fogged rapidly from the top, with a large amount of smoke.

## Description

### TECHNICAL FIELD

The present disclosure relates to the field of electronic vaporization technologies, and in particular, to a heating mechanism and an electronic vaporization device.

### BACKGROUND

An aerosol is a colloidal dispersion system formed by dispersing small solid or liquid particles and suspending the particles in a gas medium. Since the aerosol can be absorbed by a human body through a respiratory system, users are provided with a new alternative absorption manner. For example, an electronic vaporization device that can generate aerosols by baking and heating herbal or ointment aerosol-generating substrates is applied to different fields to deliver inhalable aerosols to the users, replacing conventional product forms and absorption manners.

The electronic vaporization device heats an aerosol-generating substrate by using a heating assembly, to generate an aerosol for uses to smoke. For an electronic vaporization device adopting electromagnetic heating, a heating assembly thereof includes an electromagnetic heating coil and a heating element. The electromagnetic heating coil is energized to generate a magnetic field, and the heating element is located in the magnetic field generated by the electromagnetic heating coil to heat up. The aerosol-generating substrate is in contact with the heating element, and the heating element heats and vaporizes the aerosol-generating substrate.

However, the conventional electronic vaporization device adopting electromagnetic heating has the problem of slow fogging, which leads to bad user experience.

### SUMMARY

Based on the above, there is a need to provide a heating mechanism and an electronic vaporization device that can speed up fogging with respect to the problem of slow fogging with the conventional electronic vaporization device.

According to an aspect of the present disclosure, a heating mechanism is provided. The heating mechanism includes: a heating element configured in a longitudinal shape, the heating element being provided with an accommodating cavity extending along its longitudinal direction; and an electromagnetic heating coil arranged around the heating element. In the longitudinal direction of the heating element, the axial center of the electromagnetic heating coil is misaligned with the longitudinal center of the accommodating cavity.

In the heating mechanism, when the electromagnetic heating coil is energized, the heating element is heated by using the principle of electromagnetic induction, and then an aerosol-generating substrate accommodated in the heating element is heated to form an aerosol for a user to inhale. Moreover, in the longitudinal direction of the heating element, the axial center of the electromagnetic heating coil is misaligned with the longitudinal center of the accommodating cavity. After the electromagnetic heating coil is energized, a heating electric field with the strongest magnetic induction can be formed at a position corresponding to its own axial center. The heating element has the maximum heat at a position corresponding to the axial center of the electromagnetic heating coil, and the position is misaligned with the longitudinal center of the accommodating cavity in the longitudinal direction, which means that the maximum heat is located on one side of the longitudinal center of the accommodating cavity. For an aerosol-generating substrate in the accommodating cavity, the high-temperature field can be distributed to the top, so that the aerosol-generating substrate can be fogged rapidly from the top, with a large amount of smoke.

In an embodiment, the heating element has a high-temperature zone and a medium-temperature zone arranged along its longitudinal direction, and the axial center of the electromagnetic heating coil is located in the high-temperature zone.

In an embodiment, the center of the accommodating cavity along its longitudinal direction is located on a boundary line between the high-temperature zone and the medium-temperature zone.

In an embodiment, the heating element has a low-temperature zone, the medium-temperature zone is connected between the low-temperature zone and the high-temperature zone, and an orthographic projection of the electromagnetic heating coil towards the heating element covers the high-temperature zone and the medium-temperature zone and is misaligned with the low-temperature zone.

In an embodiment, in the longitudinal direction of the heating element, a misalignment height between the axial center of the electromagnetic heating coil and the longitudinal center of the accommodating cavity ranges from 3.5 mm to 4.5 mm.

In an embodiment, the accommodating cavity is configured to accommodate an aerosol-generating substrate with a length of 20 mm, the axial length of the electromagnetic heating coil is 18 mm, and the top of the electromagnetic heating coil is 3 mm higher than that of the aerosol-generating substrate.

In an embodiment, the electromagnetic heating coil has a plurality of sub-coils in an axial direction, and the plurality of sub-coils are wound so as to be spaced evenly apart from each other.

In an embodiment, the heating mechanism further includes a mounting rack, the electromagnetic heating coil is arranged around the mounting rack, and the heating element is arranged in the mounting rack. The mounting rack has a positioning groove extending helically along the axial direction, and the electromagnetic heating coil is embedded in the positioning groove.

According to another aspect of the present disclosure, an electronic vaporization device is provided. The electronic vaporization device includes the heating mechanism according to the above aspect.

In an embodiment, the electronic vaporization device further includes a mouthpiece fitted on the heating mechanism, and the axial center of the electromagnetic heating coil is misaligned towards an upper side of the mouthpiece relative to the longitudinal center of the accommodating cavity.

### BRIEF DESCRIPTION OF THE DRAWINGS

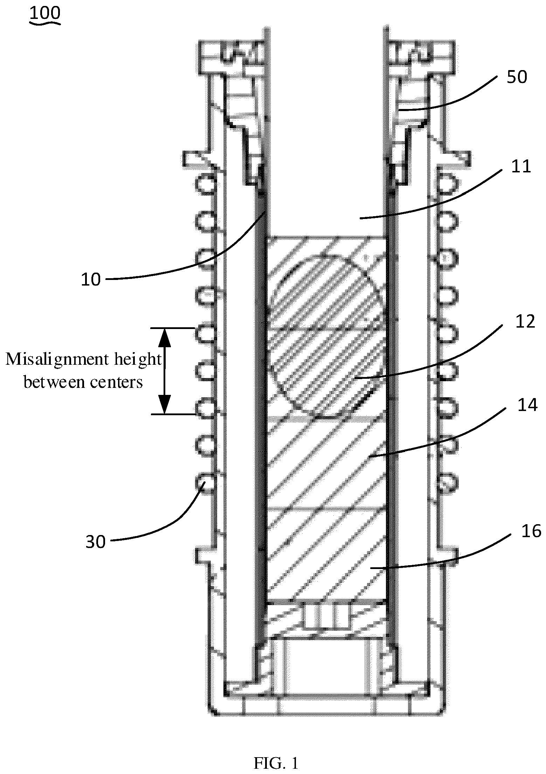

FIG. 1 is a schematic structural diagram illustrating a heating mechanism according to an embodiment of the present disclosure.

### Reference signs:

100: heating mechanism; 10: heating element; 11: accommodating cavity; 12: high-temperature zone; 14: medium-temperature zone; 16: low-temperature zone; 30: electromagnetic heating coil; and 50: mounting rack.

### DETAILED DESCRIPTION OF THE EMBODIMENTS

In order to make the above objectives, features, and advantages of the present disclosure more obvious and understandable, specific implementations of the present disclosure are described in detail below with reference to the accompanying drawings. In the following description, many specific details are set forth in order to fully understand the present disclosure. However, the present disclosure can be implemented in many other ways different from those described herein, and those skilled in the art can make similar improvements without departing from the connotation of the present disclosure. Therefore, the present disclosure is not limited by the specific implementation disclosed below.

In the description of the present disclosure, it should be understood that the orientation or position relationships indicated by the terms "central", "longitudinal", "transverse", "length", "width", "thickness", "up", "down", "front", "back", "left", "right", "vertical", "horizontal", "top", "bottom", "inner", "outer", "clockwise", "counterclockwise", "axial", "radial", "circumferential", etc. are based on the orientation or position relationships shown in the accompanying drawings and are intended to facilitate the description of the present disclosure and simplify the description only, rather than indicating or implying that the device or element referred to must have a particular orientation or be constructed and operated in a particular orientation, and therefore are not to be interpreted as limiting the present disclosure.

In addition, the terms "first" and "second" are used for descriptive purposes only, which cannot be construed as indicating or implying a relative importance or implicitly specifying the number of the indicated technical features. Thus, the features defined with "first" and "second" may explicitly or implicitly include at least one feature. In the description of the present disclosure, "a plurality of" means at least two, such as two or three, unless specifically stated otherwise.

In the present disclosure, unless otherwise specifically stated and limited, the terms "mount," "join," "connect", "fix", etc. should be understood in a broad sense, such as, a fixed connection, a detachable connection, or an integral connection; a mechanical connection, or an electrical connection; a direct connection, an indirect connection through an intermediate medium, internal communication between two elements, or an interaction of two elements, unless otherwise expressly defined. For those of ordinary skill in the art, the specific meanings of the foregoing terms in the present disclosure can be understood on a case-by-case basis.

In the present disclosure, unless otherwise explicitly specified and defined, a first feature being "on" or "under" a second feature may be a case that the first feature is in direct contact with the second feature, or the first feature is in indirect contact with the second feature via an intermediate medium. Furthermore, the first feature being "over", "above" and "on top of" the second feature may be a case that the first feature is directly above or obliquely above the second feature, or only means that the level of the first feature is higher than that of the second feature. The first feature being "below", "underneath" or "under" the second feature may be a case that the first feature is directly underneath or obliquely underneath the second feature, or only means that the level of the first feature is lower than that of the second feature.

It should be noted that when one element is referred to as "fixed to" or "arranged on" another element, it may be directly disposed on the another element or an intermediate element may exist. When one element is considered to be "connected to" another element, it may be directly connected to the another element or an intermediate element may co-exist. The terms "vertical", "horizontal", "up", "down", "left", "right" and similar expressions used herein are for illustrative purposes only, and do not represent unique embodiments.

Referring to FIG. 1, FIG. 1 is a schematic structural diagram illustrating a heating mechanism 100 according to an embodiment of the present disclosure. The heating mechanism 100 according to this embodiment of the present disclosure includes a heating element 10 and an electromagnetic heating coil 30.

The heating element 10 is provided with an accommodating cavity 11 extending along its longitudinal direction. The accommodating cavity 11 is configured to accommodate an aerosol-generating substrate. The electromagnetic heating coil 30 is arranged around the heating element 10. When the electromagnetic heating coil 30 is energized, the heating element 10 is heated by using the principle of electromagnetic induction, and then the aerosol-generating substrate accommodated in the heating element 10 is heated to form an aerosol for a user to inhale.

Moreover, in the longitudinal direction of the heating element 10, the axial center of the electromagnetic heating coil 30 is misaligned with the longitudinal center of the accommodating cavity 11. After the electromagnetic heating coil 30 is energized, a heating electric field with the strongest magnetic induction can be formed at a position corresponding to its own axial center. The heating element 10 has the maximum heat at a position corresponding to the axial center of the electromagnetic heating coil 30, and the position is misaligned with the longitudinal center of the accommodating cavity 11 in the longitudinal direction, which means that the maximum heat is located on one side of the longitudinal center of the accommodating cavity 11. For the aerosol-generating substrate in the accommodating cavity 11, the high-temperature field can be distributed to the top, so that the aerosol-generating substrate can be fogged rapidly from the top, with a large amount of smoke.

Further, the heating element 10 has a high-temperature zone 12 and a medium-temperature zone 14 arranged along its longitudinal direction, and the axial center of the electromagnetic heating coil 30 is located in the high-temperature zone 12. In other words, the heating element 10 is divided into a high-temperature zone 12 and a medium-temperature zone 14 in its longitudinal direction, and the axial center of the electromagnetic heating coil 30 is in a range where the high-temperature zone 12 is located, so that the position of the strongest magnetic induction of the electromagnetic heating coil 30 is shifted to the high-temperature zone 12, and the top of the aerosol-generating substrate in the heating element 10 is located in the high-temperature zone 12. The top of the aerosol-generating substrate can be first heated and vaporized by using the high-temperature zone 12, thereby enabling rapid fogging.

It may be understood that the heating mechanism 100 is applied to the electronic vaporization device. When the user uses the electronic vaporization device, airflow flows along a direction from the medium-temperature zone 14 to the high-temperature zone 12, and the aerosol-generating substrate located in the high-temperature zone 12 is subjected to a higher temperature, which may be first vaporized and then flow out with the airflow, for the user to inhale.

Further, the center of the accommodating cavity 11 along its longitudinal direction is located on a boundary line between the high-temperature zone 12 and the medium-temperature zone 14. In other words, the high-temperature zone 12 and the medium-temperature zone 14 are defined in a way such that the axial center of the electromagnetic heating coil 30 is located above the center of the accommodating cavity 11 along its longitudinal direction, the high-temperature zone 12 is located above the center of the accommodating cavity 11 along its own longitudinal direction, and at least a part below the center of the accommodating cavity 11 along its own longitudinal direction is the medium-temperature zone 14.

In some embodiments, the heating element 10 further has a low-temperature zone 16, the medium-temperature zone 14 is connected between the low-temperature zone 16 and the high-temperature zone 12, and an orthographic projection of the electromagnetic heating coil 30 towards the heating element 10 covers the high-temperature zone 12 and the medium-temperature zone 14 and is misaligned with the low-temperature zone 16. In other words, the electromagnetic heating coil 30 is arranged around the high-temperature zone 12 and the medium-temperature zone 14 but is not arranged around the low-temperature zone 16. In this way, the electromagnetic heating coil 30 is raised so that the axial center of the electromagnetic heating coil 30 moves up to the high-temperature zone 12.

Meanwhile, the low-temperature zone 16, the medium-temperature zone 14, and the high-temperature zone 12 are sequentially arranged along the longitudinal direction of the heating element 10 to form a high, medium, and low-temperature gradient field. When the heating mechanism 100 starts heating, the electromagnetic heating coil 30 causes the high-temperature zone 12 of the heating element 10 to have the maximum heat, locally heats the aerosol-generating substrate for rapid fogging, and simultaneously preheats the medium-temperature zone 14 and the low-temperature zone 16. After completion of the vaporization in the high-temperature zone 12, the medium-temperature zone 14 intervenes, followed by the low-temperature zone 16, to finally complete the vaporization of the aerosol-generating substrate as a whole. In this way, the high, medium and low gradient temperature distribution enables the aerosol-generating substrate to be fogged rapidly and continuously, vaporization consistency is better, and the aerosol-generating substrate can be uniformly vaporized.

In some embodiments, in the longitudinal direction of the heating element 10, a misalignment height between the axial center of the electromagnetic heating coil 30 and the longitudinal center of the accommodating cavity 11 ranges from 3.5 mm to 4.5 mm. In this way, the axial center of the electromagnetic heating coil 30 is moved up by 3.5 mm to 4.5 mm to effectively increase a temperature of an upper part of the heating element 10 to form the high-temperature zone 12. Optionally, in the longitudinal direction of the heating element 10, the misalignment height between the axial center of the electromagnetic heating coil 30 and the longitudinal center of the accommodating cavity 11 is 4 mm.

Further, the accommodating cavity 11 is configured to accommodate an aerosol-generating substrate with a length of 20 mm, the axial length of the electromagnetic heating coil 30 is 18 mm, and the top of the electromagnetic heating coil 30 is 3 mm higher than that of the aerosol-generating substrate. The aerosol-generating substrate and the electromagnetic heating coil 30 are designed with the above dimensions, so that the electromagnetic heating coil 30 is 3 mm higher than the aerosol-generating substrate, and then the axial center of the electromagnetic heating coil 30 is 4 mm higher than the longitudinal center of the aerosol-generating substrate, so as to form the high-temperature zone 12 on the top of the aerosol-generating substrate, speeding up the fogging.

In some embodiments, the heating mechanism 100 further includes a mounting rack 50, the electromagnetic heating coil 30 is arranged around the mounting rack 50 helically, and the heating element 10 is arranged in the mounting rack 50. In this way, it is easy to assemble and fix the heating element 10 and the electromagnetic heating coil 30.

The mounting rack 50 has a positioning groove extending helically along the axial direction, and the electromagnetic heating coil 30 is embedded in the positioning groove. Specifically, the positioning groove is a profiling groove manufactured by imitating a shape of the electromagnetic heating coil 30, so that the electromagnetic heating coil 30 is firmly fixed to the mounting rack 50.

Optionally, the heating assembly further includes a magnetic shield arranged outside the electromagnetic heating coil 30. On the one hand, the magnetic shield can fix the electromagnetic heating coil 30, and the magnetic shield can prevent radiation of electromagnetic waves to the outside by the electromagnetic heating coil 30. Specifically, the magnetic shield is bonded and fixed to the electromagnetic heating coil 30.

In any one of the above embodiments, the electromagnetic heating coil 30 has a plurality of sub-coils in an axial direction, and the plurality of sub-coils are wound so as to be spaced evenly apart from each other. That is, pitches between any two adjacent sub-coils in the electromagnetic heating coil 30 are identical, so as to evenly and helically wind the electromagnetic heating coil 30, so that a magnetic field formed by the electromagnetic heating coil 30 is evenly distributed in the axial direction, the strongest magnetic induction of the electromagnetic heating coil 30 is located at the axial center of the electromagnetic heating coil 30, and the high-temperature zone 12 at the top of the heating element 10 can be formed by raising the electromagnetic heating coil 30.

Further, the electromagnetic heating coil 30 is formed by extending helically in the axial direction through at least one wire bundle, and each wire bundle includes at least two wires. That is, each wire bundle includes at least two wires, and each wire bundle is twisted by at least two wires.

Specifically in this embodiment, the heating mechanism 100 includes a heating element 10 and an electromagnetic heating coil 30. The heating element 10 is provided with an accommodating cavity 11 extending along its longitudinal direction. The accommodating cavity 11 is configured to accommodate an aerosol-generating substrate. The electromagnetic heating coil 30 is arranged around the heating element 10. In the longitudinal direction of the heating element 10, the axial center of the electromagnetic heating coil 30 is misaligned with the longitudinal center of the accommodating cavity 11, and the heating element 10 is provided with a high-temperature zone 12, a medium-temperature zone 14, and a low-temperature zone 16 arranged along its longitudinal direction. The medium-temperature zone 14 is connected between the high-temperature zone 12 and the low-temperature zone 16. An orthographic projection of the electromagnetic heating coil 30 towards the heating element 10 covers the high-temperature zone 12 and the medium-temperature zone 14 and is misaligned with the low-temperature zone 16. In other words, the electromagnetic heating coil 30 is arranged around the high-temperature zone 12 and the medium-temperature zone 14 but is not arranged around the low-temperature zone 16. In this way, the electromagnetic heating coil 30 is raised so that the axial center of the electromagnetic heating coil 30 moves up to the high-temperature zone 12.

Meanwhile, the low-temperature zone 16, the medium-temperature zone 14, and the high-temperature zone 12 are sequentially arranged along the longitudinal direction of the heating element 10 to form a high, medium, and low-temperature gradient field. When the heating mechanism 100 starts heating, the electromagnetic heating coil 30 causes the high-temperature zone 12 of the heating element 10 to have the maximum heat, locally heats the aerosol-generating substrate for rapid fogging, and simultaneously preheats the medium-temperature zone 14 and the low-temperature zone 16. After completion of the vaporization in the high-temperature zone 12, the medium-temperature zone 14 intervenes, followed by the low-temperature zone 16, to finally complete the vaporization of the aerosol-generating substrate as a whole. In this way, the high, medium and low gradient temperature distribution enables the aerosol-generating substrate to be fogged rapidly and continuously, vaporization consistency is better, and the aerosol-generating substrate can be uniformly vaporized.

In an embodiment of the present disclosure, an electronic vaporization device is further provided, including the above heating mechanism 100. The heating mechanism 100 includes a heating element 10 and an electromagnetic heating coil 30.

The heating element 10 is provided with an accommodating cavity 11 extending along its longitudinal direction. The accommodating cavity 11 is configured to accommodate an aerosol-generating substrate. The electromagnetic heating coil 30 is arranged around the heating element 10. When the electromagnetic heating coil 30 is energized, the heating element 10 is heated by using the principle of electromagnetic induction, and then the aerosol-generating substrate accommodated in the heating element 10 is heated to form an aerosol for a user to inhale.

Moreover, in the longitudinal direction of the heating element 10, the axial center of the electromagnetic heating coil 30 is misaligned with the longitudinal center of the accommodating cavity 11. After the electromagnetic heating coil 30 is energized, a heating electric field with the strongest magnetic induction can be formed at a position corresponding to its own axial center. The heating element 10 has the maximum heat at a position corresponding to the axial center of the electromagnetic heating coil 30, and the position is misaligned with the longitudinal center of the accommodating cavity 11 in the longitudinal direction, which means that the maximum heat is located on one side of the longitudinal center of the accommodating cavity 11. For the aerosol-generating substrate in the accommodating cavity 11, the high-temperature field can be distributed to the top, so that the aerosol-generating substrate can be fogged rapidly from the top, with a large amount of smoke.

In some embodiments, the electronic vaporization device further includes a mouthpiece (not shown). The mouthpiece is fitted on the heating mechanism 100, and the axial center of the electromagnetic heating coil 30 is misaligned towards an upper side of the mouthpiece relative to the longitudinal center of the accommodating cavity 11.

The mouthpiece is a component that the user directly contacts when inhaling. The user generally holds the mouthpiece in the mouth and inhales into the mouthpiece. In this case, the aerosol generated by heating the aerosol-generating substrate in the electronic vaporization device can be inhaled. "The axial center of the electromagnetic heating coil 30 is misaligned towards an upper side of the mouthpiece relative to the longitudinal center of the accommodating cavity 11" means that the electromagnetic heating coil 30 is raised towards the upper side of the mouthpiece, so as to move the axial center of the electromagnetic heating coil 30 up towards the upper side of the mouthpiece to quickly heat the aerosol-generating substrate in the accommodating cavity 11 to get close to the top of the mouthpiece, speeding up the fogging.

Further, the heating element 10 has a high-temperature zone 12 and a medium-temperature zone 14 arranged along its longitudinal direction, and the axial center of the electromagnetic heating coil 30 is located in the high-temperature zone 12. In other words, the heating element 10 is divided into a high-temperature zone 12 and a medium-temperature zone 14 in its longitudinal direction, and the axial center of the electromagnetic heating coil 30 is in a range where the high-temperature zone 12 is located, so that the position of the strongest magnetic induction of the electromagnetic heating coil 30 is shifted to the high-temperature zone 12, and the top of the aerosol-generating substrate in the heating element 10 is located in the high-temperature zone 12. The top of the aerosol-generating substrate can be first heated and vaporized by using the high-temperature zone 12, thereby enabling rapid fogging.

Specifically, the center of the accommodating cavity 11 along its longitudinal direction is located on a boundary line between the high-temperature zone 12 and the medium-temperature zone 14. In other words, the high-temperature zone 12 and the medium-temperature zone 14 are defined in a way such that the axial center of the electromagnetic heating coil 30 is located above the center of the accommodating cavity 11 along its longitudinal direction, the high-temperature zone 12 is located above the center of the accommodating cavity 11 along its own longitudinal direction, and at least a part below the center of the accommodating cavity 11 along its own longitudinal direction is the medium-temperature zone 14, so.

In some embodiments, the heating element 10 has a low-temperature zone 16, the medium-temperature zone 14 is connected between the low-temperature zone 16 and the high-temperature zone 12, and an orthographic projection of the electromagnetic heating coil 30 towards the heating element 10 covers the high-temperature zone 12 and the medium-temperature zone 14 and is misaligned with the low-temperature zone 16. In other words, the electromagnetic heating coil 30 is arranged around the high-temperature zone 12 and the medium-temperature zone 14 but is not arranged around the low-temperature zone 16. In this way, the electromagnetic heating coil 30 is raised so that the axial center of the electromagnetic heating coil 30 moves up to the high-temperature zone 12.

Meanwhile, the low-temperature zone 16, the medium-temperature zone 14, and the high-temperature zone 12 are sequentially arranged along the longitudinal direction of the heating element 10 to form a high, medium, and low-temperature gradient field. When the heating mechanism 100 starts heating, the electromagnetic heating coil 30 causes the high-temperature zone 12 of the heating element 10 to have the maximum heat, locally heats the aerosol-generating substrate for rapid fogging, and simultaneously preheats the medium-temperature zone 14 and the low-temperature zone 16. After completion of the vaporization in the high-temperature zone 12, the medium-temperature zone 14 intervenes, followed by the low-temperature zone 16, to finally complete the vaporization of the aerosol-generating substrate as a whole. In this way, the high, medium and low gradient temperature distribution enables the aerosol-generating substrate to be fogged rapidly and continuously, vaporization consistency is better, and the aerosol-generating substrate can be uniformly vaporized.

In some embodiments, in the longitudinal direction of the heating element 10, a misalignment height between the axial center of the electromagnetic heating coil 30 and the longitudinal center of the accommodating cavity 11 ranges from 3.5 mm to 4.5 mm. In this way, the axial center of the electromagnetic heating coil 30 is moved up by 3.5 mm to 4.5 mm to effectively increase a temperature of an upper part of the heating element 10 to form the high-temperature zone 12. Optionally, in the longitudinal direction of the heating element 10, the misalignment height between the axial center of the electromagnetic heating coil 30 and the longitudinal center of the accommodating cavity 11 is 4 mm.

Specifically, the accommodating cavity 11 is configured to accommodate an aerosol-generating substrate with a length of 20 mm, the axial length of the electromagnetic heating coil 30 is 18 mm, and the top of the electromagnetic heating coil 30 is 3 mm higher than that of the aerosol-generating substrate. The aerosol-generating substrate and the electromagnetic heating coil 30 are designed with the above dimensions, so that the electromagnetic heating coil 30 is 3 mm higher than the aerosol-generating substrate, and then the axial center of the electromagnetic heating coil 30 is 4 mm higher than the longitudinal center of the aerosol-generating substrate, so as to form the high-temperature zone 12 on the top of the aerosol-generating substrate, speeding up the fogging.

In some embodiments, the heating mechanism 100 further includes a mounting rack 50, the electromagnetic heating coil 30 is arranged around the mounting rack 50 helically, and the heating element 10 is arranged in the mounting rack 50. In this way, it is easy to assemble and fix the heating element 10 and the electromagnetic heating coil 30. The mounting rack 50 has a positioning groove extending helically along the axial direction, and the electromagnetic heating coil 30 is embedded in the positioning groove. Specifically, the positioning groove is a profiling groove manufactured by imitating a shape of the electromagnetic heating coil 30, so that the electromagnetic heating coil 30 is firmly fixed to the mounting rack 50.

In any one of the above embodiments, the electromagnetic heating coil 30 has a plurality of sub-coils in an axial direction, and the plurality of sub-coils are wound so as to be spaced evenly apart from each other. That is, pitches between any two adjacent sub-coils in the electromagnetic heating coil 30 are identical, so as to evenly and helically wind the electromagnetic heating coil 30, so that a magnetic field formed by the electromagnetic heating coil 30 is evenly distributed in the axial direction, the strongest magnetic induction of the electromagnetic heating coil 30 is located at the axial center of the electromagnetic heating coil 30, and the high-temperature zone 12 at the top of the heating element 10 can be formed by raising the electromagnetic heating coil 30.

The technical features in the above embodiments may be randomly combined. For concise description, not all possible combinations of the technical features in the above embodiments are described. However, all the combinations of the technical features are to be considered as falling within the scope described in this specification provided that they do not conflict with each other.

The above embodiments only describe several implementations of the present disclosure, and their description is specific and detailed, but cannot therefore be understood as a limitation on the patent scope of the present disclosure. It should be noted that those of ordinary skill in the art may further make variations and improvements without departing from the conception of the present disclosure, and these all fall within the protection scope of the present disclosure. Therefore, the patent protection scope of the present disclosure should be subject to the appended claims.

## Claims

1. A heating mechanism (100), comprising:
a heating element (10) configured in a longitudinal shape, the heating element (10) being provided with an accommodating cavity (11) extending along its longitudinal direction; and
an electromagnetic heating coil (30) arranged around the heating element (10);
wherein, in the longitudinal direction of the heating element (10), the axial center of the electromagnetic heating coil (30) is misaligned with the longitudinal center of the accommodating cavity (11).

2. The heating mechanism (100) according to claim 1, wherein the heating element (10) has a high-temperature zone (12) and a medium-temperature zone (14) arranged along its longitudinal direction, and the axial center of the electromagnetic heating coil is located in the high-temperature zone (12).

3. The heating mechanism (100) according to claim 2, wherein the center of the accommodating cavity (11) along its longitudinal direction is located on a boundary line between the high-temperature zone (12) and the medium-temperature zone (14).

4. The heating mechanism (100) according to claim 2, wherein the heating element (10) has a low-temperature zone (16), the medium-temperature zone (14) is connected between the low-temperature zone (16) and the high-temperature zone (12), and an orthographic projection of the electromagnetic heating coil (30) towards the heating element (10) covers the high-temperature zone (12) and the medium-temperature zone (14) and is misaligned with the low-temperature zone (16).

5. The heating mechanism (100) according to claim 1, wherein, in the longitudinal direction of the heating element (10), a misalignment height between the axial center of the electromagnetic heating coil (30) and the longitudinal center of the accommodating cavity (11) ranges from 3.5 mm to 4.5 mm.

6. The heating mechanism (100) according to claim 5, wherein the accommodating cavity (11) is configured to accommodate an aerosol-generating substrate with a length of 20 mm, the axial length of the electromagnetic heating coil (30) is 18 mm, and the top of the electromagnetic heating coil (30) is 3 mm higher than that of the aerosol-generating substrate.

7. The heating mechanism (100) according to any one of claims 1 to 6, wherein the electromagnetic heating coil (30) has a plurality of sub-coils in an axial direction, the plurality of sub-coils being wound so as to be spaced evenly apart from each other.

8. The heating mechanism (100) according to any one of claims 1 to 6, further comprising a mounting rack (50), the electromagnetic heating coil (30) is arranged around the mounting rack (50), and the heating element (11) is arranged in the mounting rack,
wherein the mounting rack (50) has a positioning groove extending helically along the axial direction, the electromagnetic heating coil (30) being embedded in the positioning groove.

9. An electronic vaporization device, comprising the heating mechanism (100) according to any one of claims 1 to 8.

10. The electronic vaporization device according to claim 9, further comprising a mouthpiece fitted on the heating mechanism (100), wherein the axial center of the electromagnetic heating coil (30) is misaligned towards an upper side of the mouthpiece relative to the longitudinal center of the accommodating cavity (11).
